(19) Europäisches Patentamt
European Patent Office
Office européen des brevets

(11) **EP 4 678 222 A1**

(12) **EUROPEAN PATENT APPLICATION**

(43) Date of publication:
**14.01.2026 Bulletin 2026/03**

(21) Application number: **24187120.1**

(22) Date of filing: **08.07.2024**

(51) International Patent Classification (IPC):
**A61N 1/08** *(2006.01)* **A61N 1/37** *(2006.01)*
**A61N 1/375** *(2006.01)*

(52) Cooperative Patent Classification (CPC):
**A61N 1/3754; A61N 1/086; A61N 1/3718**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **SCHOTT AG**
  **55122 Mainz (DE)**
- **SCHOTT Primoceler Oy**
  **33720 Tampere (FI)**

(72) Inventors:
- **THOMAS, Jens Ulrich**
  **55122 Mainz (DE)**
- **POLOJAERVI, Ville**
  **33710 Tampere (FI)**
- **MAEAETTAENEN, Antti**
  **33720 Tampere (FI)**
- **LUNDEN, Heidi**
  **33720 Tampere (FI)**
- **LAHTINEN, Ossi**
  **33720 Tampere (FI)**

(74) Representative: **Schott Corporate IP**
**Hattenbergstraße 10**
**55122 Mainz (DE)**

(54) **IMPLANTABLE DEVICE**

(57) An implantable device (1) is provided comprising a housing (2), an electronic or electrical component (4) and at least one antenna (8), wherein the housing (2) is formed by at least two non-metallic substrates (10, 20, 22) which are bonded by means of a laser bonding process, wherein at least one of the non-metallic substrates (10, 20, 22) is a glass substrate (10) and has at least one electrical feedthrough configured as a through glass via (30) comprising a conductive rod (32) electrically connecting the inside of the housing (2) to the outside through said glass substrate (10). The conductive rod (32) of the at least one through glass via (30), the at least one electronic or electrical component (4) and the at least one antenna (8) are configured and arranged such that the implantable device (1) has an interference volume I within which a relative change $\Delta f/f_0$ of an MRI signal frequency $f$ relative to the frequency of an undisturbed signal $f_0$ caused by the presence of the implantable device (1) is more than $1 \times 10^{-6}$, a ratio m of the interference volume I to a volume V of the implantable device I/V being less than 40.

**Fig. 1**

**Description**

The invention relates to an implantable device comprising a housing, an electronic or electrical component and at least one antenna, wherein the housing is formed by at least two non-metallic substrates which are bonded by means of a laser bonding process, wherein at least one of the non-metallic substrates is a glass substrate and has at least one electrical feedthrough configured as a through glass via comprising a conductive rod electrically connecting the inside of the housing to the outside through said glass substrate.

Implantable devices, such as pacemakers or cochlear implants, require a housing as enclosure to protect the components inside the enclosure from adverse environmental conditions. Also, the housing must contain any components or compounds enclosed in the housing and must prevent any leaks. The materials used for the housing must also be suitable for direct contact with tissue of the human or animal body. Typically, metal cases made of titanium are used as housings for implants.

WO 2021/078731 A1 discloses a glass compound arrangement comprising at least a base substrate and a cover substrate, which form an enclosure. The substrates are hermetically welded by means of at least one laser weld line. The arrangement may be used as enclosure for making a medical implant and may comprise through glass vias to provide an electrical connection of a function component housed inside a cavity of the enclosure and the outside of the enclosure.

However, the problem with known implantable devices is that they obscure large parts of the surrounding tissue during imaging procedures or interfere with the imaging procedures due to their mere presence. If important features or structures such as a tumor are located near such an implantable device, they could easily be overlooked during the examination with the imaging procedure.

In case of x-ray based imaging, such as conventional x-ray or computer tomography (CT), materials having a high x-ray absorption or the capability of scattering the x-ray radiation can hinder the imaging process. In case of magnet resonance imaging (MRI), any materials interfering with the magnetic field can hinder the imaging process.

While implants using a glass enclosure such as shown in WO 2021/078731 A1 in particular reduce the amount of metal material compared to implants using a metal case, there are still many components such as batteries and antennas which comprise metal and thus may cause interference with the imaging processes.

The invention therefore aims to provide an implantable that does not interfere with imaging procedures, or at least interferes as little as possible.

Disclosure of the invention

An implantable device is proposed, the implantable device comprising a housing, an electronic or electrical component and at least one antenna, wherein the housing is formed by at least two non-metallic substrates which are bonded by means of a laser bonding process, wherein at least one of the non-metallic substrates is a glass substrate and has at least one electrical feedthrough configured as a through glass via comprising a conductive rod electrically connecting the inside of the housing to the outside through said glass substrate.

The conductive rod of the at least one through glass via, the at least one electronic or electrical component and the at least one antenna are configured and arranged such that the implantable device, when it is subjected to an MRI imaging process, has an interference volume I, within which a relative change $\Delta f/f_0$ of an MRI signal frequency $f$ relative to the frequency of an undisturbed signal $f_0$ caused by the presence of the implantable device is more than $1 \cdot 10^{-6}$, wherein a ratio m of the interference volume I to a volume V of the implantable device I/V is less than 40, preferably less than 20 and more preferably less than 10.

Magnetic resonance imaging (MRI) is a volumetric imaging technique which uses strong magnetic fields, magnetic field gradients and radio waves to generate volumetric images, in particular of the human or animal body. During the imaging procedure, the used radio waves are in resonance with a nuclear magnetic spin of protons in hydrogen nuclei, wherein the resonance frequency or signal frequency $f_0$ is dependent on the strength of the magnetic field. For a clear and undisturbed image, a homogeneous magnetic field is required. The presence of the implantable device may in particular disturb the homogeneity of the used magnetic field which causes distortions in the acquired images.

Accordingly, the implantable device is configured to minimize any influence on a magnetic field into which the implantable device is introduced. Minimizing this influence on the disturbance of a magnetic field thus reduces the interference volume I for MRI imaging. The interference volume I for magnetic resonance imaging (MRI) volumetric imaging is defined as the volume in which a deviation of the magnetic field due to the implantable device is more than 1 ppm ($1 \cdot 10^{-6}$). As the resonance frequency is proportional to the magnetic field, this can be detected by a relative change $\Delta f/f_0$ of the detected MRI radio frequency (RF) signal frequency $f$ relative to the undisturbed signal frequency $f_0$ for the undisturbed magnetic field by more than 1 ppm.

The volume V is defined as the volume occupied by the implantable device. The interference volume I should be as small as possible and should ideally not be much larger than the volume V occupied by the implantable device itself. Accordingly, the ratio m of the interference volume I to the volume V of the implantable device I/V should be less than 40, preferably less than 20 and more preferably less than 10.

In order to optimize the ratio m of the interference volume I to the volume V of the implantable device, the used materials are selected carefully and/or the antenna, the at least one electronic or electrical component and/or the through glass vias are placed inside or on the housing such that any interference that is caused is preferably located within the volume V occupied by the implant.

The selection of non-metallic materials for the substrates, such as glass, is favorable as metallic materials can disturb the magnetic field or can shield RF signals. Additionally, substrate materials may be selected which are transparent for laser radiation used in a laser welding process for bonding the substates together. The laser bonding process allows for a mechanically stable and fluid tight and in particular hermetic bond without the need for additional sealing materials or sealing structures. This allows a reduction in overall size and/or mass of the housing of the implantable device compared to conventional housings made of metal. The reduction of size and mass also contributes to a reduction of the overall interference of the implantable device with imaging procedures.

In order to limit any occlusion or interference with imaging methods, it is preferred to design the implantable device to be as small as possible. The volume of the device is preferably less than 4 cm$^3$, more preferably less than 1 cm$^3$ and the largest linear dimension of the implantable device is preferably less than 5 cm.

Further, in order to reduce the overall size of the implantable device, it is preferred to reduce the thickness of the walls of the housing. Preferably, a maximum wall thickness of the housing is equal to or less than 1 mm, more preferably 500 µm or less, most preferably 200 µm or less. As the substrates may in particular form a bottom wall or a top wall of the housing, it is preferred that the thickness of a substrate of the housing is equal to or less than 1 mm, more preferably 500 µm or less, most preferably 200 µm or less.

In a preferred laser bonding process, laser bond lines are introduced at or near the interface between two neighboring substrates, wherein in a mixing zone, material of the two substrates is mixed to form a strong bond. The mixing zone extends preferably into both of the substrates. No additional material is required to promote adhesion so that the interface between two neighboring substrates is free from any adhesives or bonding agents.

In such a preferred laser bonding process, a short-pulsed laser beam from a laser source, for which at least one of the substrates is transparent, is focused to a spot inside the formed substrate stack. By choosing the repetition rate and a scan rate of the laser, the individual laser pulses are arranged so closely together that a resulting nonlinear absorption zone of a laser pulse within the material is in contact with a neighboring nonlinear absorption zone of a further laser pulse, or even overlaps with it, such that heat accumulation can occur. Due to the accumulated heat, the material of the substrate stack is locally melted and a continuous welding "line" or laser bond line can be obtained. For creating such a continuous welding line, a focus plane of the laser beam is arranged close to but not at the interface between the two substrates. The laser beam is arranged at a distance below the interface between the two substrates such that the accumulated heat causes the material of substrates to be bonded to locally melt and mix so that a hermetic bond is formed.

The through-glass vias comprise a conductive rod which is electrically conductive and provides an electrical connection from one side of a substrate to the respective other side of the substrate. The conductive rod may be arranged in a through opening of the substrate.

Preferably, the material of the conductive rod is selected from a non-ferromagnetic conductive material, such as a non-ferromagnetic metal or a doped semiconductor material. The conductive material preferably comprises tungsten, titanium, platinum, gold, silver, copper, doped silicon, and combinations of said conductive materials.

The through glass vias may be covered on one or both sides with a coating. Preferably, the coating is structured to form an electric contact pad or a conductive conductor trace. A contact pad may in particular be configured as a solder pad for attachment of a wire via a solder material or may be configured as a connector pad to be contacted by e.g. a plug or connector.

The coating may comprise one or more layers and may in particular include one or more of an adhesion layer, a diffusion barrier layer and a contact layer. Preferably, at least for a coating arranged on an outward facing surface of the housing, and at least for the outermost layer in case of a multi-layer structure, corrosion resistant materials are selected for the coating.

As the coating is electrically conductive, each of the layers in case of a multi-layer structure is selected from an electrically conductive material.

A thickness of the adhesion layer is preferably in the range of from 2 nm to 200 nm, more preferably from 10 nm to 150 nm and most preferably from 20 nm to 100 nm.

The diffusion barrier layer is chosen such that diffusion of materials from the corrosion resistant contact layer or substances from outside of the hermetic enclosure cannot diffuse or propagate towards the conductive rods and vice versa. In particular, the diffusion barrier layer is chosen such that materials contained in a solder material or an adhesive material as well as oxygen from the environment cannot damage the conductive rods of the electrical feedthroughs. Further the material of the diffusion barrier layer is preferably chosen to be biocompatible. Biocompatible materials are non-toxic and have not injurious effects on biological systems.

Preferably, the diffusion barrier layer is made from or comprises platinum (Pt), titan-nitride (TiN) and combinations thereof. If the diffusion barrier layer is the outermost layer of the multilayer structure, the material of the diffusion barrier layer is preferably chosen from a biocompatible material.

A thickness of the diffusion barrier layer is preferably in the range of from 20 nm to 200 nm, more preferably from 40 nm to 150 nm and most preferably from 50 nm to 100 nm.

The corrosion resistant contact layer is preferably not only resistant to corrosive environments but is preferably also a material with good electrical conductivity and good wettability for solder materials in order to enable high quality electrical connections. Still further, as the corrosion resistant contact layer preferably forms the outermost layer of the electrically conductive coating, the corrosion resistant contact layer is preferably chosen from a biocompatible material.

Preferably, the corrosion resistant contact layer is made from or comprises gold (Au) or a platinoid or a platinoid-oxide, such as Iridium (Ir), in particular Iridium-Oxide ($IrO_2$).

A thickness of the corrosion resistant contact layer is preferably in the range of from 50 nm to 200nm, more preferably from 80 nm to 150 nm and most preferably from 75 nm to 100 nm.

Preferably, the material of the glass substrate and/or the electrically conductive coating are selected such that said materials are resistant to exposure to an NaCl solution in water, in particular to a solution of 700 g/l NaCl. The housing may, for example, be immersed in such a solution for seven days at a temperature of 37°C and may then be examined visually for signs of corrosion. Further, it is possible to assess the corrosion resistance by determining a loss of mass. A material may then be considered to be corrosion resistant if the loss of mass is less than 5%, preferably less than 3% and most preferably less than 1%. The total mass of the electrically conductive coating is a small quantity so that a loss of mass of the material of the electrically conducting coating is hard to measure. However, if the coating is not resistant, it will develop gaps after exposure with a corrosive environment and said gaps will lead to an exposure of the conductive rods to the NaCl solution. Accordingly, the material of the electrically conductive coating is in particular considered to be corrosion resistant, if the conductive rods remain protected and thus an overall mass loss of the material of the conductive rods after exposure of the enclosure to an NaCl solution in water with 700g/l NaCl at a temperature of 37 °C for 7 days, is preferably less than 5%, more preferably less than 3% and most preferred less than 1%. Further, for the material of the glass substrate it is preferred that the mass loss of the material of the glass substrate is less than 5%, preferably less than 3% and more preferably less than 1%.

As the loss of mass of a conductive rod is a small quantity, it is preferred to use an ensemble of several enclosures to test for corrosion resistance. For example, if the housing has Nv conductive rods of mass $m_r$, an ensemble of N housings is used so that the total mass of the conduction rods is larger than 0.1 g:

$$M_r = N \cdot N_V \cdot m_r > 0.1 \text{ g}.$$

The measurement may then be performed by first drying the samples, for example in in a drying cabinet using IR-drying at 100°C for 30 min. After drying, the initial weight $M_0$ of the dry ensemble is determined, for example by means of an analytical balance (e.g., VWRI 611-3350 / LA314i from Avantor). The ensemble of housings is then immersed in NaCl -solution (700 g/l) for seven days at 37°C. After the testing period, the samples are rinsed with de-ionzed water and then dried, for example in a drying cabinet (IR-drying, 100°C for 30 min). After drying, the weight $M_1$ of the dry ensemble is determined, for example by means of an analytical balance (e.g., VWRI 611-3350 / LA314i from Avantor).

Hence, $\Delta M = M0 - M1$ and $\Delta M/M_r < 5\%, < 3\%$, most preferred less than 1%.

In order to discriminate, whether the loss in mass results from loss of mass of the conductive rods or from a corrosion of the glass (which should also be avoided), the test may be repeated on a matching ensemble using the same method with glass housings of the same spatial dimensions and of the same glass but with no though glass vias and thus without conductive rods. A difference in the determined mass losses $\Delta M$ between these two measurements yields the mass loss of the material of the conductive rods.

The material of the conductive rods, in particular in case a metal is chosen, can cause local changes to the strength of the magnetic field when the implantable device is subjected to an MRI imaging process. It has been found that it is advantageous to limit those disturbances to the volume which is occupied by the implantable device and thus not of interest for the imaging process. Accordingly, it is preferred to arrange the conductive rod of the at least one through glass via in a central area of the glass substrate. The central area is preferably defined as the area of the glass substrate having a distance to an edge of the glass substrate which is larger than 1/5, preferably larger than 1/4, more preferably larger than 1/3 of the smallest lateral dimension of the glass substrate. The lateral directions are the directions perpendicular to the thickness direction of the substrate. Additionally or alternatively, the central area may be defined as the area having a distance of at least 2 mm, preferably at least 5 mm, more preferably at least 10 mm from the edges of the glass substrate.

Preferably, a diameter of the conductive rod is less than 500 $\mu$m, preferably less than 250 $\mu$m, more preferably less than 100 $\mu$m, even more preferably less than 50 $\mu$m, most preferably less than 1 $\mu$m. A smaller diameter of the conductive rod results in less conductive material required for the conductive rod. Preferably, the total mass of the conductive rods of the through glass vias of the implantable device is less than 15 mg, preferred less than 6 mg, most preferred less than 3 mg.

Of particular interest for avoiding any disturbance of a magnetic field caused by the implantable device are ferromagnetic materials. Preferably, the implantable device comprises no or less than 5000 mg, preferably less than 1000 mg, most preferred less than 500 mg of ferromagnetic materials. Ferromagnetic materials include, for example, Cobalt, Nickel, Iron, Gadolinium, Dysprosium and Chromium-Dioxide.

The implantable device is preferably also configured to reduce a negative impact on x-ray based imaging processes, such as conventional 2D x-ray imaging and computer tomography (CT) volumetric imaging.

In case of CT imaging, an apparent size of the implantable device could be larger than the actual size, e.g. due to scattering of x-rays or absorption of x-rays. This larger apparent size leads to an occluded volume which is larger than the actual volume V occupied by the implantable device. Preferably, the conductive rod of the at least one through glass via, the at least one electronic or electrical component and/or the at least one antenna are configured and arranged such that for CT volumetric imaging a volume occluded by the implantable device is less than M V, wherein V is the volume of the implantable device and M is less than 2, preferably less than 1.5.

Preferably, the volume enclosed by the housing comprises a function volume in which the at least one electronic or electrical component and/or the at least one antenna are located, and a window volume, wherein any material within the window volume is non-ferromagnetic and has an absorption for x-ray radiation given by the attenuation coefficient for X-ray photons of 59.4 keV (tungsten K-alpha line) of less than $0.5 \text{ cm}^{-1}$, preferably less than $0.45 \text{ cm}^{-1}$, more preferably $0.4 \text{ cm}^{-1}$, and wherein a ratio of the function volume to the window volume is in the range of from 0.02 to 0.98, preferred from 0.05 to 0.8, most preferred from 0.1 to 0.5.

When a two-dimensional x-ray image of a human or animal body is prepared, a projection of a three-dimensional volume is made. As at least some parts of the implantable device absorb x-ray photons, a projection of the implantable device will be visible in the image in form of a shadow. The area occupied by this shadow is also referred to as x-ray occlusion area. The exact size and shape of the image of the implantable device varies depending on the direction of projection. The part of the projection of the implantable device corresponding to the functional volume will be occluded in the image and is thus referred to as x-ray occlusion area. As the window volume of the implantable device does not absorb x-ray photons, the shadow or x-ray occlusion area does not comprise the projection of the window volume of the implantable device, allowing to see features and structures of the human and animal body in the image in the projection area of the window volume.

Preferably, the implantable device has a first diagnostic imaging orientation for which an x-ray occlusion area is less than 50%, preferred 20%, most preferred 10% of the total area of the projection of the implantable device along the first diagnostic imaging direction.

The implantable device is typically not shaped as a perfect sphere and thus the size and shape of the projection of the implantable device is dependent on the direction of projection. Preferably, the implantable device has a shape such that for a second diagnostic imaging orientation, the implantable device has a projected cross-section area which is at most 50 % of the area of the largest projected cross-section area of the implantable device.

In combination with a preferred imaging direction of the respective human or animal body, this can be exploited to orientate the implantable device such that the occlusion in the prepared x-ray images is reduced by aligning the first or second diagnostic imaging direction with the preferred imaging direction.

In order to further improve the properties of the implantable device with respect to the different medical imaging methods, it is preferred to reduce the mass and number of components inside the implantable device as much as possible.

Preferably, the implantable device is free from batteries. Batteries, require a large volume within the housing of the implantable device and may comprise materials which are magnetic, in particular ferromagnetic, and/or absorb x-ray photons. Instead of the use of batteries, it is preferred to include an antenna for wireless power transmission. In addition to such an antenna, the implantable device may comprise a capacitor, in particular a super capacitor for storage of electrical energy.

Preferably, any voids or cavities inside the housing of the implantable device are filled with an inert gas or are evacuated, preferably to a residual pressure of less than 700 mbar, more preferably less than 500 mbar, most preferably less than 300 mbar, so that they are essentially free from water moisture.

The avoidance of moisture within the housing helps to ensure the longevity of the implantable device, in particular of any electronic or electrical component housed within the housing of the implantable device. Arrangement of a moisture absorbing material within the housing may further assist in ensuring that the voids and cavities remain essentially free of water vapor and moisture over the entire lifespan of the implantable device.

Preferably at least one of the substrates is a glass substrate that includes a surface area that has been activated to serve as moisture absorber. This avoids the need of an additional element for the absorption of moisture. The activation of a glass substrate or a part of a glass substrate may be achieved e.g. by fire polishing, dry etching, dry cleaning or plasma cleaning. An enclosure having such an activated substrate is disclosed in WO 2023/078658 A1.

The housing is preferably configured such that a hermetic enclosure is formed, wherein a cavity or function zone is enclosed within the housing. Hermetic sealing of the housing ensures in particular that no moisture leaks into the housing and that any inert gas is contained inside the housing. Also, any substances which may be harmful to the human or animal body are safely contained inside the hermetic enclosure and vice versa any component contained inside the hermetic enclosure is protected from environmental influences. As used herein, hermetically sealed means in particular that the formed housing has a helium leakage rate of less than $1\cdot10^{-8}$ mbar·l/sec and is preferably in the range $1\cdot10^{-10}$ mbar·l/sec to $1.10^{-9}$ mbar·l/sec.

The implantable device comprises at least one antenna. The one or more antennas may be configured to be used for wireless power transmission and/or for data communication. Wireless power transmission allows in particular to avoid the need for batteries within the housing to power the electronic or electrical component. If a battery or a capacitor is still required, as wireless power transmission is not always available when a device within the implantable device is to be powered, the size of such a battery or capacitor may be reduced as the battery or capacitor may be easily recharged without having to remove the implantable device from within the human or animal body.

Antennas may be placed directly on a substrate of the housing or directly adjacent to the housing as the housing material is non-metallic and non-conducting. Thus, the dimensions of an antenna structure may fully utilize the inner dimensions of a cavity within the housing.

Preferably, at least one antenna is formed as a wire or coil structure that is printed onto one of the substrates using a conductive material or is formed by a structured conductive coating of one of the substrates. Preferably, the antenna is located on an inside facing surface of the respective substrate.

Such an antenna in form of a conductive material or coating on a surface of a substrate requires only a thin layer of conductive material and thus requires only a small amount of conductive material. Any interference of such a thin film antenna with a medical imaging process is thus reduced. For producing such an antenna, the coating may be printed by means of a conductive ink or may be applied to a surface of a substrate and then structured by selective removal or modification of the coating. The structuring can, for example, be performed by using a photolithographic process wherein a photomask is produced to structure the coating by selective etching or performing a lift-off process wherein a certain part of the coating is removed. Alternatively, a laser process may be used to remove or modify a coating on a surface of a substrate. Preferably, the same laser which is used to perform the laser bonding process is also used for selective removal or modification of the coating. Modification may be performed by selective heating of the coating. Removal of the coating may be performed by laser ablation or melting of the coating and the adjacent part of the substrate, thus effectively removing the coating from the surface of the substrate. The conductive coating may be selected from any electrically conductive material, such as a conductive metal. The electrically conductive coating may be chosen to be light transparent, in particular if the electrically conductive coating is applied to a transparent glass substrate. Indium tin oxide (ITO) may be used as transparent electrically conductive coating.

In order to protect such a thin film antenna from outside influences, it is preferred to arrange a thin film antenna or structured conductive coating on an inside facing surface of a substrate forming the housing. As the non-metallic substrates are transparent for radio waves, the function of the antennas is not hindered by the housing. However, it is possible to arrange the thin film antenna or structured coating on an outside facing side. In this case, it is preferred to protect the structured coating by applying a corrosion resistant coating layer.

Additionally or alternatively, the at least one antenna is formed as a coil of conductive wire. A wire-coil is preferably constructed using a non-ferromagnetic metal wire such as aluminum, silver, gold, titanium, or tungsten wire. Also, non-ferromagnetic alloys of said metals may be used. The outer shape of the coil is preferably adapted to the shape of a cavity of the housing and is preferably dimensioned such that the coils dimensions are maximized. The coil may be arranged without any gap to a wall of the cavity. Within the antenna coil, a window volume may be located which is free of x-ray absorbing material.

Preferably, the at least one electronic or electrical component is an optoelectronic component and a part of at least one of the non-metallic substrates is configured as an optical window which is transparent for light which is emitted and/or received by the optoelectronic component. The respective part of the substrate is preferably coated with an electrically conductive coating. The coating may in particular be in electrical connection with one or more of the through glass vias. The ability of designing the housing such that it is transparent not only for radio frequency signals, but also for electromagnetic radiation, in particular in the visible spectrum of about 450 nm to 750 nm and/or in the near infrared spectrum of about 750 nm to 1300 nm, allows the easy integration of optical methods for measurements or interaction with the human or animal body.

Preferably, the housing is formed by exactly two substrates, wherein at least one of the substrates comprises a cavity.

Alternatively, it is preferred to form the housing using a base substrate, a cover substrate and an intermediate substrate, wherein the intermediate substrate comprises at least one opening which forms a cavity within the housing. It is also possible to include more than one intermediate substrate and/or to include one or more further substrates, for example to form a larger cavity or to form several cavities. If several cavities are formed, the cavities may be interconnected by means of through glass vias located in one or more of the further substrates.

Preferably, the material of the glass substrate is a glass material or a glass ceramic material. A preferred glass material is a borosilicate glass, such as the borosilicate glass available under the designation borofloat33 from SCHOTT AG.

Preferably, the material of a further non-metallic substrate is a glass material, a glass ceramic material, a ceramic material, silicon, sapphire, or a polymer. Suitable polymer materials include polyetheretherketone (PEEK,) polyethylene, polypropylene, acrylonitrile butadiene styrene (ABS) and polycarbonate.

A preferred glass material for the further substrate is a borosilicate glass available under the designation borofloat33 from SCHOTT AG. Another example would be fused silica (e.g. Heraeus Suprasil® 1), AF32, D263T eco, MEMPAX, 45S5 Bioglass®, a boroaluminosilicate glass such as EAGLE XG®.

The implantable device may further comprise a sleeve which at least partially, preferably completely, surrounds the housing of the implantable device. This sleeve is preferably made from a resilient soft material such as medical grade silicone rubber.

The outer shape of the implant is preferably free from sharp edges and includes preferably only rounded edges having a radius of curvature of at least 0.05 mm, more preferably 0.1 mm, most preferably at least 0.5 mm

A substrate or a part of a substrate forming the housing may be configured to serve as optical window. This optical window may include an anti-reflection coating. Other areas of the housing may include coatings to form opaque or reflective areas. Preferably, the glass substrate having the through glass vias is configured to have such an optical window.

The implantable device described herein is preferably configured as a pacemaker, cardiac monitor, neuro-stimulator, retina implant, neural interface, cochlear implant, strain-sensor for fracture fixation or joint replacement implants.

It is understood that the above-mentioned features and those to be explained below can be used not only in the respective combination as shown, but also in other combinations or on their own, without leaving the scope of the present invention.

Preferred embodiments of the invention are shown in the figures and will be explained in more detail in the following description, wherein identical reference numerals refer to identical or similar components or elements.

Brief description of the figures:

The figures show in schematic form

Figure 1 a schematic cross-section view of an example implant,

Figure 2 the implant of figure 1 embedded in tissue and powered by means of an external power transmitter,

Figure 3 a schematic top view of the implant of figure 1,

Figure 4 a schematic depiction of an image obtained via a volumetric imaging process,

Figure 5 a schematic depiction of an image obtained via a 2D imaging process,

Figure 6 a reference MRI image,

Figure 7 an MRI image of an empty glass housing,

Figure 8 an MRI image of a glass housing populated with components to form an implantable device,

Figure 9 an MRI image of a prior art titanium housing.

Figure 10 a top view of housing having a structured coating,

Figure 11 an enlarged cross-sectional view of the structured coating of figure 10.

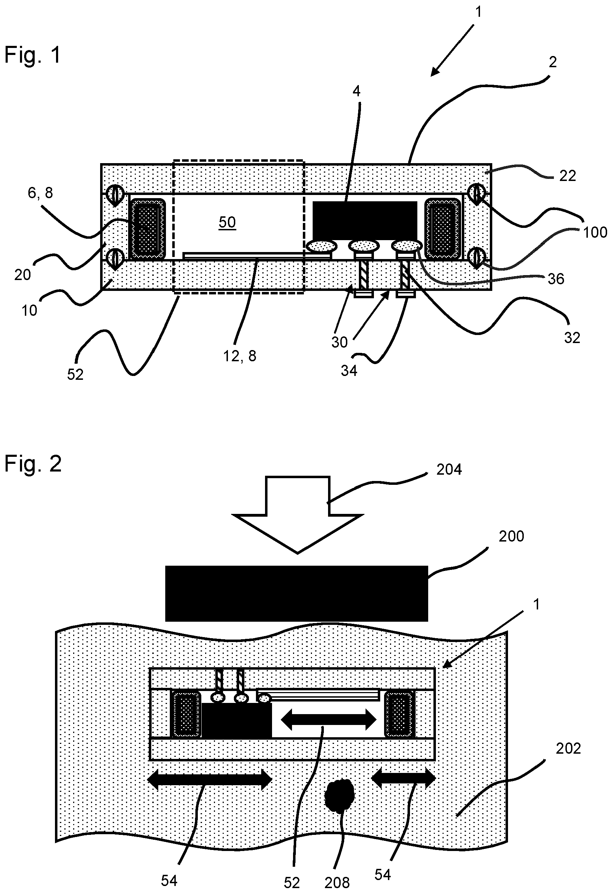

Figure 1 shows a schematic cross-section view of an example implant 1 having a housing 2. The housing 2 has in this example three substrates 10, 20, 22, wherein the respective neighboring substrates 10, 20, 22 in the formed stack are bonded together by means of laser weld lines 100. A glass substrate 10 forms a bottom of a cavity 50 enclosed by the housing 2, a second substrate 20 forms sidewalls of the cavity 50 and a further substrate 22 forms a cover of the cavity 50 enclosed by the housing 2. As the substrates 10, 20, 22 which form the housing 2 are laser welded together without the need for any further materials, such as adhesives, seals, or connection means such as screws, the housing 2 can be designed to be compact. As the thickness of the substrates 10, 20, 22 as well as the thickness of the side walls of the cavity 50 can also be chosen to be low, e.g. in the range of from 200 $\mu$m to 1000 $\mu$m, the size and outer shape of the implant is essentially given by the size and shape of the cavity 50.

The glass substrate 10 has through glass vias 30 which allow an electrical connection between the outside and an electronic or electrical component 4 located inside the cavity 50 of the housing 2. In the schematic view of figure 1, two through glass vias 30 are visible. On an outside facing side and on an inside facing side of the glass substrate 10, the through glass vias 30 are covered by contact pads 34. The contact pads 34 provide corrosion protection of the through glass vias 30 and provide a reliable contact point for forming an electrical contact.

The implant 1 of figure 1 further comprises two antennas 8, wherein one is configured as a wire coil 6 and another is configured as a structured coating 12 located on an inside facing surface of the glass substrate 10. In other examples, the implant 1 could be configured to include only a single antenna 8 or more than two antennas 8. The structured coating 12 is electrically conductive and shaped as a loop to form a thin film antenna. In the schematic view of figure 1, a solder connection 36 is depicted to indicate an electrical connection between the electronic or electrical component 4 and the structured coating 12. Further solder connections 36 are depicted to indicate an electrical connection between the electronic or electrical component 4 and the through glass vias 30. The wire coil 6 is also electrically connected to the electronic or electrical component 4, but this connection is not depicted in the cross-section view of figure 1.

As can be seen in figure 1, the through glass vias 30 are spaced apart from side walls of the cavity 50. This helps to confine most of the possible disturbances of a magnetic field to a volume located within the implantable device 1. Further, it can be seen that the components of the implant 1 having a significant x-ray absorption, in particular the wire coil 6 and the electronic or electrical component 4, are grouped together to allow for a window volume 52 within the implantable device 1 which is essentially free from x-ray absorbing materials.

Figure 2 schematically depicts the implant 1 of figure 1 embedded in tissue 202 of a human or animal body. A power transmitter 200 is arranged outside of the human or animal body to electrically power the implant 1 and in particular to power the electronic or electrical component 4. This can be achieved, for example, by inductive coupling of a transmitting antenna of the power transmitter 200 with the wire coil 6 of the implant 1. By providing power from outside of the implant 1, a battery-less design is possible. This is advantageous as batteries are relatively large and contain materials which may interfere with x-ray or MRI imaging methods.

As can be seen from figure 2, the volume V of the implantable device 1 can be separated into an occluded volume 54, which is occupied by the wire coil 6 and the electronic or electrical component 4, and a window volume 52 which is essentially free from materials which absorb x-ray radiation. For example, if an x-ray image is prepared with the viewing direction or projection direction aligned with a first diagnostic direction 204 of the implantable device 1, x-ray radiation may pass through the window volume of 52 the implantable device 1 unhindered, allowing to image features 208 of the human or animal body, such as a tumor, which would otherwise have been obscured by the implantable device 1.

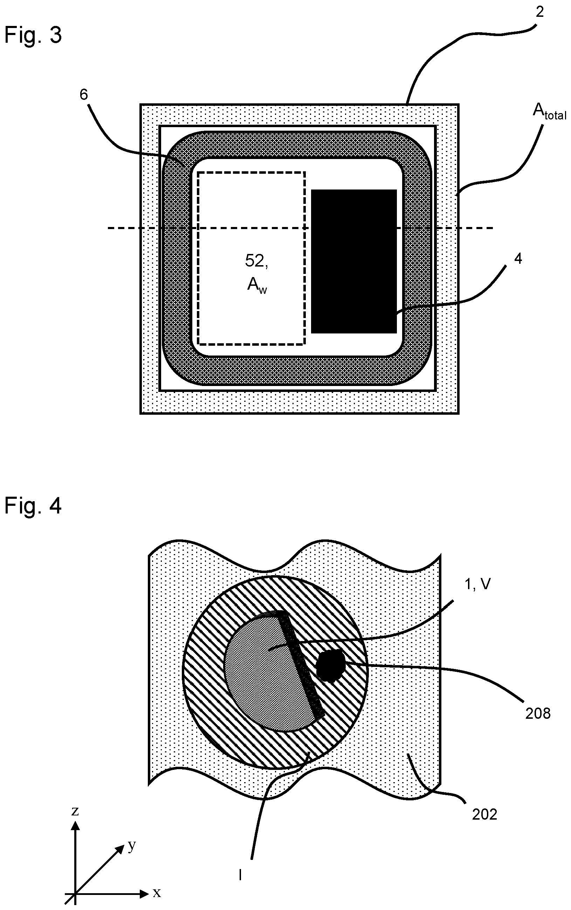

Figure 3 shows a schematic top view of the implantable device 1 of figure 1. In this example, the housing 2 of the implantable device 1 is a box having a square-shape. In other examples, the housing 2 could have a different shape, for example, the housing 2 could be shaped like a disc or a cylinder.

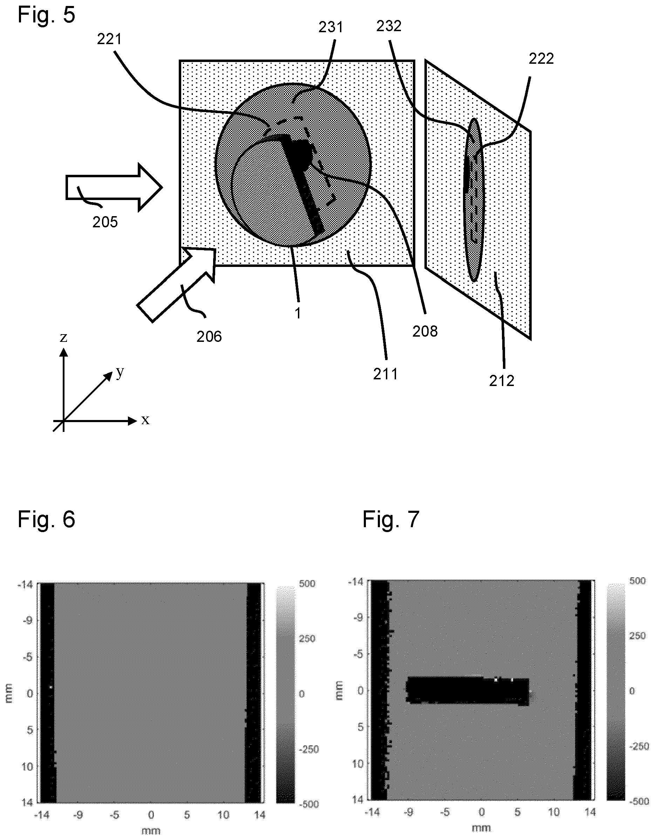

Figures 4 and 5 depict an implantable device 1 having a housing 2 in the shape of a circular segment which is embedded in tissue 202 of a human or animal body. Inside the tissue, there is a feature 208, for example a tumor, which is to be imaged using a medical imaging process.

Figure 4 shows a schematic depiction of an image obtained via a volumetric imaging process, for example magnetic resonance imaging (MRI). Magnetic resonance imaging (MRI) is a volumetric imaging technique which uses strong magnetic fields, magnetic field gradients and radio waves to generate the volumetric image. During the imaging procedure, the used radio waves are in resonance with a nuclear magnetic spin of protons in hydrogen nuclei, wherein the resonance frequency or signal frequency $f_0$ is dependent on the strength of the magnetic field. For a clear and undisturbed image, a homogeneous magnetic field is required. The presence of the implantable device 1 disturbs the homogeneity of the used magnetic field in a volume which is herein referred as interference volume I. These disturbances of the magnetic field cause distortions in the acquired image and obscure the feature 208 which is in the depicted example located within the interference volume I. The interference volume I is larger than a volume V which is occupied by the implantable device 1. By choosing the material and placement of the conductive rod 32 of the at least one through glass via 30, the at least one electronic or electrical component 4 and the at least one antenna 8, the size of the interference volume I is reduced.

Figure 5 shows a schematic depiction of 2D images obtained via a 2d imaging process such as x-ray imaging. X-ray radiation is sent along a direction 205, 206 and an image of the shadow of the human or animal body including the implantable device 1 is obtained. In figure 5, a first projected image 211 is obtained along a second preferred diagnostic direction 205 and a second projection image 212 is obtained along a further direction 206.

The example implantable device 1 shown in figure 5 has a shape of a circular segment and has a large cross-section when viewed from a direction perpendicular to the plane of the circular-segment shape, and a smaller cross-section when viewed from a direction parallel to the plane of the circular-segment shape. Accordingly, in the first projected image 211, the implantable device 1 has a first cross section area 221 which is larger than a second cross section area 222 in the second projected image 222. Accordingly, in order to image features 208, such as a tumor, which are located close to the implantable device 1, it is preferably to perform the 2D imaging process along a direction parallel to the second preferred diagnostic direction 205. Further, the implantable device 1 is preferably inserted into the human or animal body in an orientation such that the second preferred diagnostic direction 205 corresponds to the usually used imaging direction for the respective region or organ of the human or animal body.

Figures 6, 7, 8 and 9 each show a two-dimensional slice of a volumetric MRI image. MRI imaging was carried out at a magnetic field of 3 T using a pre-clinical imaging system (MR Solutions, UK). During imaging, samples were placed inside quadrature $^1$H volume coils at room temperature.

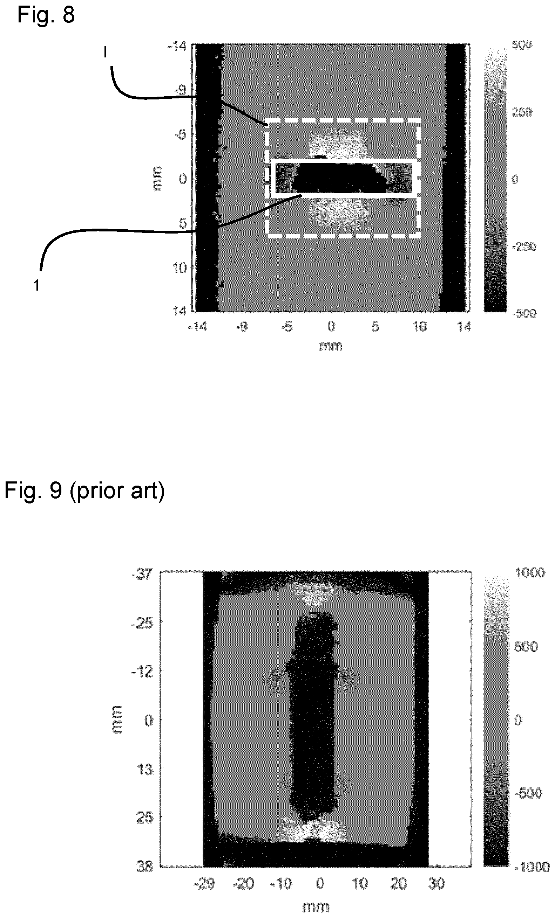

MRI images were prepared to asses the disturbance of the magnetic field due to the presence of the sample implants. The x and y axis denote the 2D coordinates of the two-dimensional slice of the volumetric image and the grayscale denotes the deviation of the obtained frequency $f$ of the nuclear spin resonance signal frequency from the undisturbed signal frequency $f_0$ of 128 MHz. The undisturbed signal frequency $f_0$ for the proton in hydrogen nuclei depends on the strength of the magnetic field and is thus an indicator for the homogeneity of the magnetic field. For figures 6, 7 and 8, the scale for the signal frequency change is from -500 Hz to +500 Hz. For figure 9, the scale for the signal frequency change is from -1000 Hz to + 1000 Hz.

The sample preparation consisted of plating the bottom of a test tube with melted 8% gelatin, placing the sample on top of cooled gelatin and filling the remainder of the test tube with gelatin. Imaging was started after an overnight solidification period. For reference, one tube containing only 8% gelatin was also prepared.

Figure 6 shows a reference MRI image of a test tube filled with a reference gel. The reference gel is 8% gelatin in water. As can be seen from figure 6, the volume occupied by the reference gel appears homogeneous in the grayscale image, indicating a homogeneous and undisturbed magnetic field within the MRI imaging device.

Figure 7 shows an MRI image of an empty glass housing 2 having the dimensions 14.5 mm × 13.5 mm × 3.7 mm. The housing 2 is clearly visible as a dark shadow in the image as the housing 2 displaces the reference gel and thus the hydrogen nuclei generating the MRI signal. Furthermore, it can be seen that the area surrounding the empty glass housing 2 appears homogeneous, indicating that the empty glass housing 2 is not disturbing the magnetic field within the MRI imaging device.

Figure 8 shows an MRI image of an implantable device 1, wherein a glass housing 2 comprising through glass vias 30 has been populated with a printed circuit board comprising electronic components 4. Further, the through glass vias 30 are provided with a coating to provide contact pads 34. The printed circuit board was connected to the through glass vias 30 via solder. The housing 2 of the implantable device has the dimensions 13.8 mm × 8.3 mm × 3.5 mm.

As can be seen from the grayscale, the MRI signal frequency and thus the local magnetic field is distorted by the metal materials present in the implantable device 1. Due to the distortions, the signal frequency within an interference volume I is changed by a factor of more than +/-1 · 10^{-6} of the undisturbed frequency f0 of 128 MHz and thus by more than +/- 128 Hz in absolute numbers. The dashed-lines in figure 8 mark a boundary box which contains all distortions in excess of +/- 128 Hz.

Figure 9 shows an MRI image of a prior art titanium housing. The housing has the dimensions 50 × 50 × 10 mm. Due to the larger size of titanium housing and size constraints of the test tubes, the orientation of the titanium housing was changed compared to the previous samples.

As can be seen from the grayscale of figure 9, the absolute strength of the distortion of the titanium housing alone is already stronger than for the complete implant having the glass housing.

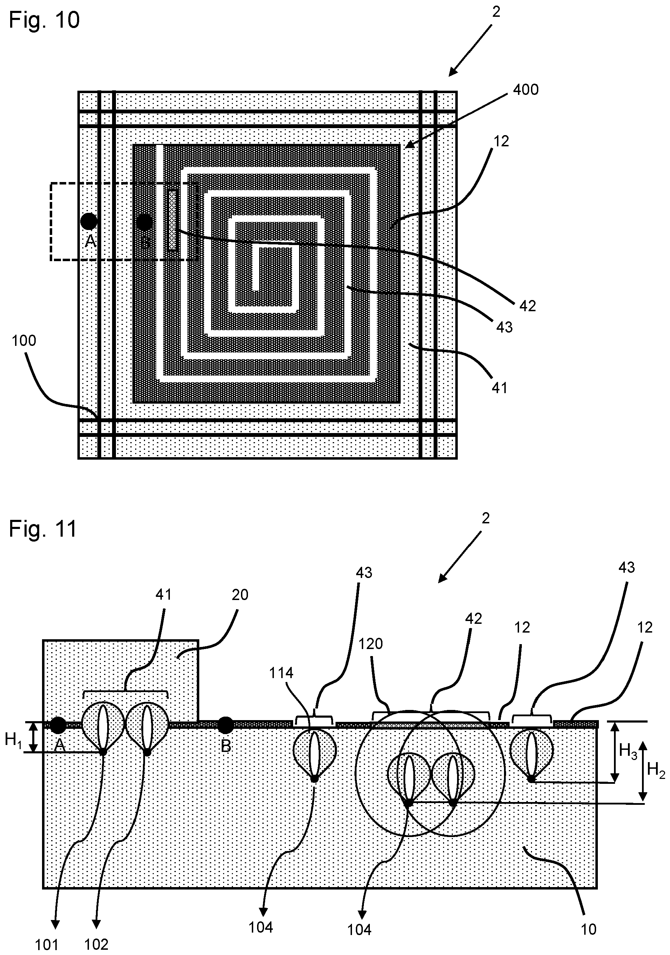

Figure 10 shows a top view of a housing 2 having a structured coating 12.

The housing 2 depicted in figure 10 comprises a substrate stack having a first substrate 10 and a second substrate 20. The coating 12 located on the side of the first substrate 10 facing towards the second substrate 20 is an electrically conductive coating such as an indium tin oxide (ITO) coating. The materials of the substrates 10, 20 are electrically non-conductive.

In a first laser treated zone 41 comprising the weld lines 100, the coating 12 has been effectively removed so that there is no conductive path from an area located inside a space defined by the weld lines 100 to the outside. For example, there is no continuity between a point A located outside the defined space and a point B located inside the defined space. The remaining coating 12 within the defined space has been structured in a coil-like manner by preparing a third laser treated zone 43, in which the coating 12 is effectively removed, but no bond between the first substrate 10 and the second substrate 20 is formed.

Further, it is possible to influence the electrical resistance of the coating 12 by heat treatment within a second laser treated zone 42. The coating 12 within said second laser treated zone 42 is not removed, but modified by heat so that the electrical conductivity is adjusted.

Figure 11 shows an enlarged cross-sectional view of the structured coating of figure 10.

From the side view of figure 11 it can be seen that the second substrate 20 is configured as a spacer defining side walls and is not covering the entire surface of the first substrate 10 with the coating 12. In a contact area, where the first substrate 10 with coating 12 is in direct contact with the second substrate 20, two weld lines 100 are arranged in parallel, a first weld line 101 and a second weld line 102. The heat introduced by the two weld lines 100 forms a first laser treated zone 41 in which material of the first substrate 10, the coating 12 and the second substrate 20 is molten and mixes to form a hermetic bond between the two substrates 10, 20. The material of the coating 12 is part of the material mixture, which effectively removes the coating 12 from the surface of the first substrate 10. As can be seen in figure 11, the focus plane for forming the first laser treated zone 41 is located at a first depths $H_1$. This depth is chosen such that the molten zone 114 created by the laser reaches from the lower first substrate 10 into the upper second substrate 20.

In a second laser treated zone 42, the coating 12 is not removed, but its properties are altered by the heat affected zones 120 caused by additional laser lines 104 located at a second depth $H_2$ from the surface of the first substrate 10. In case of a conductive ITO coating as coating 12, the electrical resistance is increased within the second laser treated zone 42 so that a resistor is formed by the selective laser treatment.

In a third laser treated zone 43, the coating 12 is effectively removed without bonding the first substrate 10 to the second substrate 20. This is achieved by forming a third laser treated zone 43 in which the melted zone of a further laser weld line 104, arranged at a third depth $H_3$, touches the surface of the first substrate 10 and thus causes melting of the coating 12. The molten material of the coating 12 within said third laser treated zone 43 mixes with molten material of the first substrate 10 and is thus effectively removed from the surface of the first substrate 10. As the first substrate 10 is in this case electrically insulating, a non-conductive structure is thereby formed.

Although the present invention has been described with reference to preferred examples of embodiments, it is not limited thereto but can be modified in a variety of ways.

List of reference numerals

| | |
|---|---|
| 1 | implantable device |
| 2 | housing |
| 4 | electronic or electrical component |
| 6 | coil |
| 8 | antenna |
| 10 | glass substrate |
| 12 | coating of glass substrate |
| 20 | second substrate |
| 22 | further substrate |
| 30 | through glass via |
| 32 | conductive rod |
| 34 | contact pad |
| 36 | solder |
| 41 | first laser treated zone |
| 42 | second laser treated zone |
| 43 | third laser treated zone |
| 50 | cavity, enclosed volume |
| 52 | window volume |
| 54 | occluded volume |

| | |
|---|---|
| $A_w$ | window area |
| $A_{total}$ | total area |
| 100 | weld line |
| 101 | first weld line |
| 102 | second weld line |
| 104 | additional laser line |
| 110 | absorption zone |
| 112 | elongated bubble |
| 114 | melted zone |
| 120 | heat affected zone |
| 200 | power transmitter |
| 202 | body tissue |
| 204 | first preferred diagnostic direction |
| 205 | second preferred diagnostic direction |
| 206 | further direction |
| 208 | feature of interest |
| 211 | first projected image |
| 212 | second projected image |
| 221 | first cross section area |
| 222 | second cross section area |
| 231 | first obscured area |
| 232 | second obscured area |
| I | interference volume |
| V | Volume of implantable device |

**Claims**

1. Implantable device (1) comprising a housing (2), an electronic or electrical component (4) and at least one antenna (8), wherein the housing (2) is formed by at least two non-metallic substrates (10, 20, 22) which are bonded by means of a laser bonding process, wherein at least one of the non-metallic substrates (10, 20, 22) is a glass substrate (10) and has at least one electrical feedthrough configured as a through glass via (30) comprising a conductive rod (32) electrically connecting the inside of the housing (2) to the outside through said glass substrate (10), **characterized in that**

   the conductive rod (32) of the at least one through glass via (30), the at least one electronic or electrical component (4) and the at least one antenna (8) are configured and arranged such that
   the implantable device (1) has an interference volume I within which a relative change $\Delta f/f_0$ of an MRI signal frequency $f$ relative to the frequency of an undisturbed signal $f_0$ caused by the presence of the implantable device (1) is more than $1 \times 10^{-6}$, a ratio m of the interference volume I to a volume V of the implantable device I/V being less than 40.

**2.** Implantable device (1) according to claim 1, wherein the material of the conductive rod (32) is selected from a non-ferromagnetic conductive material, wherein the non-ferromagnetic conductive material preferably comprises comprise tungsten, titanium, platinum, gold, silver, copper, doped silicon, and combinations of said conductive materials, and/or wherein an electric contact pad (34) covers the through glass via (30).

**3.** Implantable device (1) according to claim 1 or 2, wherein the conductive rod (32) of the at least one through glass via (30) is arranged in a central area of the glass substrate (10), the central area being defined as the area of the glass substrate (10) having a distance to an edge of the glass substrate (10) which is larger than 1/5, preferably 1/4, more preferably 1/3 of the smallest lateral dimension of the glass substrate (10) or is defined as the area having a distance of at least 2 mm, preferably 5 mm, more preferably 10 mm from the edges of the glass substrate (10).

**4.** Implantable device (1) according to any one of claims 1 to 3, wherein a diameter of the conductive rod (32) is less than 500 $\mu$m, preferably less than 250 $\mu$m, more preferably less than 100 $\mu$m, even more preferably less than 50 $\mu$m, most preferably less than 1 $\mu$m.

**5.** Implantable device (1) according to any one of claims 1 to 4, wherein the implantable device (1) comprises no or at most 5000 mg, preferably less than 1000 mg, more preferably than 500 mg of ferromagnetic materials and/or wherein the total mass of the conductive rods (32) of the through glass vias (30) is less than 15 mg.

**6.** Implantable device (1) according to any one of claims 1 to 5, wherein the conductive rod (32) of the at least one through glass via (30), the at least one electronic or electrical component (4) and the at least one antenna (8) are configured and arranged such that for CT volumetric imaging a volume occluded by the implantable device is less than M V, wherein V is the volume of the implantable device and M is less than 2, preferably less than 1.5.

**7.** Implantable device (1) according to any one of claims 1 to 6, wherein the volume enclosed by the housing (2) comprises a function volume in which the at least one electronic or electrical component (4) and/or the at least one antenna (8) are located, and a window volume (52), wherein any material within the window volume (52) is non-ferromagnetic and has an absorption for x-ray radiation given by the attenuation coefficient for X-ray photons of 59.4 keV (tungsten K-alpha line) of less than 0.5 / cm, preferably less than 0.45 /cm, more preferably 0.4 / cm and wherein a ratio of the function volume to the window volume (52) is in the range of from 0.1 to 0.6 and/or wherein the implantable device (1) has a first diagnostic imaging orientation (204) for which an x-ray occlusion area is less than 50%, preferably less than 20%, most preferred less than 10 % of the area of the projection of the implantable device (1) along the first diagnostic imaging direction (204).

**8.** Implantable device (1) according to any one of claims 1 to 7, wherein, for a second diagnostic imaging orientation (205), the implantable device (1) has a projected cross-section area which is at most 50 % of the area of the largest projected cross-section area of the implantable device (1).

**9.** Implantable device (1) according to any one of claims 1 to 8, wherein the implantable device (1) is free from batteries.

**10.** Implantable device (1) according to any one of claims 1 to 9, wherein

voids or cavities inside the housing (2) of the implantable device (1) are filled with an inert gas or are evacuated, preferably to a residual pressure of less than 700 mbar, so that they are essentially free from water moisture and/or wherein at least one of the substrates (10, 20, 22) includes a surface area that has been activated to serve as moisture absorber.

**11.** Implantable device (1) according to any one of claims 1 to 10, wherein the at least one antenna (8) is formed as a wire or coil structure that is printed onto one of the substrates (10, 20, 22) using a conductive material or is formed by a structured conductive coating (12) of one of the substrates (10, 22, 22), wherein the antenna (8) is preferably located on an inside facing surface of the respective substrate (10, 20, 22).

**12.** Implantable device (1) according to any one of claims 1 to 11, wherein the at least one antenna (8) is formed as a coil (6) of conductive wire, wherein an outer shape of the antenna (8) preferably corresponds to a shape of a cavity of the housing (2).

**13.** Implantable device (1) according to any one of claims 1 to 12, wherein the at least one electronic or electrical component (4) is an optoelectronic component and a part of at least one of the non-metallic substrates (10, 20, 22) is configured as an optical window which is transparent for light which is emitted and/or received by the optoelectronic component.

**14.** Implantable device (1) according to any one of claims 1 to 13, wherein the housing (2) is formed by exactly two substrates, wherein at least one of the substrates comprises a cavity (50) or wherein the housing (2) is formed by a base substrate, a cover substrate and an intermediate substrate, wherein the intermediate substrate comprises at least one opening which forms a cavity (50) within the housing (2).

15. Implantable device (1) according to any one of claims 1 to 14, wherein the material of the glass substrate (10) is a glass material or a glass ceramic material, in particular a borosilicate glass or fused silica and/or the material of a further non-metallic substrate is a glass material, a glass ceramic material, a ceramic material, silicon.

16. Implantable device (1) according to any one of claims 1 to 15, wherein a maximum wall thickness of the housing (2) is less than 1 mm, preferably less than 500 $\mu$m, more preferably less than 200 $\mu$m.

17. Implantable device (1) according to any one of claims 1 to 16, wherein the implantable device (1) is configured as a pacemaker, cardiac monitor, neuro-stimulator, retina implant, neural interface, cochlear implant, or strain-sensor.

Fig. 1

Fig. 2

Fig. 3

Fig. 4

Fig. 5

221  231  232  222

205

206

1  208

211  212

z
y
x

Fig. 6                    Fig. 7

Fig. 8

Fig. 9 (prior art)

Fig. 10

2

400

12

42

43

41

100

Fig. 11

2

41    20

43

43

120

42

12

12

114

$H_1$

A         B

$H_3$    $H_2$

101    102

104

104

10

## EUROPEAN SEARCH REPORT

**Application Number**

EP 24 18 7120

### DOCUMENTS CONSIDERED TO BE RELEVANT

| Category | Citation of document with indication, where appropriate, of relevant passages | Relevant to claim |
|---|---|---|
| A | US 2017/095667 A1 (YAKOVLEV ANATOLY [US] ET AL) 6 April 2017 (2017-04-06) * the whole document * ----- | 1-17 |
| A | US 2007/268201 A1 (SAMPSELL JEFFREY B [US] ET AL) 22 November 2007 (2007-11-22) * the whole document * ----- | 1-17 |
| A | US 9 968 794 B2 (MEDTRONIC INC [US]) 15 May 2018 (2018-05-15) * the whole document * ----- | 1-17 |
| A | US 2015/101841 A1 (RUBEN DAVID A [US] ET AL) 16 April 2015 (2015-04-16) * the whole document * ----- | 1-17 |

**CLASSIFICATION OF THE APPLICATION (IPC)**

INV.
A61N1/08
A61N1/37
A61N1/375

**TECHNICAL FIELDS SEARCHED (IPC)**

A61N

The present search report has been drawn up for all claims

| Place of search | Date of completion of the search | Examiner |
|---|---|---|
| Munich | 17 October 2024 | Sopelana Martínez, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 7120

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2024

| Patent document cited in search report | Publication date | Patent family member(s) | Publication date |
|---|---|---|---|
| US 2017095667 A1 | 06-04-2017 | NONE | |
| US 2007268201 A1 | 22-11-2007 | EP 1960818 A2 | 27-08-2008 |
| | | US 2007268201 A1 | 22-11-2007 |
| | | US 2013176518 A1 | 11-07-2013 |
| | | WO 2007139651 A2 | 06-12-2007 |
| US 9968794 B2 | 15-05-2018 | CN 107106852 A | 29-08-2017 |
| | | EP 3237060 A1 | 01-11-2017 |
| | | US 2016184593 A1 | 30-06-2016 |
| | | US 2018263132 A1 | 13-09-2018 |
| | | WO 2016106269 A1 | 30-06-2016 |
| US 2015101841 A1 | 16-04-2015 | CN 105611966 A | 25-05-2016 |
| | | EP 3055019 A1 | 17-08-2016 |
| | | US 2015101841 A1 | 16-04-2015 |
| | | US 2020062633 A1 | 27-02-2020 |
| | | WO 2015054485 A1 | 16-04-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021078731 A1

- WO 2023078658 A1